Europäisches Patentamt

European Patent Office

Office européen des brevets

(11) Publication number: **0 542 301 A1**

# EUROPEAN PATENT APPLICATION

(21) Application number: **92119465.0**

(22) Date of filing: **13.11.92**

(30) Priority: **15.11.91 JP 326749/91**

(43) Date of publication of application:
**19.05.93 Bulletin 93/20**

(84) Designated Contracting States:
**DE FR GB**

(51) Int. Cl.⁵: **G01N 33/543**, G01N 33/566,
G01N 33/569, G01N 33/80

(71) Applicant: **OLYMPUS OPTICAL CO., LTD.**
**43-2, 2-chome, Hatagaya Shibuya-ku**
**Tokyo 151(JP)**

(72) Inventor: **Tamai, Toyohiro c/o Intellectual**
**Property & Legal**
**Department Olympus Optical Co., Ltd.**
**2-3, Kuboyama-cho, Hachioji-shi**
**Tokyo(JP)**

(74) Representative: **KUHNEN, WACKER &**
**PARTNER**
**Alois-Steinecker-Strasse 22 Postfach 1553**
**W-8050 Freising (DE)**

(54) A method of fixing coated biological substances by drying them.

(57) First, wheat germ lectin (WGA) is coated to the bottom of each well in a U-bottomed plate. Erythrocyte is coated to the well bottom in a single layer to prepare a plate for coating erythrocyte. Next, to each well of this plate, a drying solution containing saccharose of 20 wt%, bovine serum albumin of 0.2 wt%, and sodium dithionite of 0.05 wt% is dispensed in 100 $\mu\ell$ portions. After the plate was allowed to stand for three minutes, the drying solution was discarded and water is then removed. The plate for fixing erythrocyte was then air-dried by a dry incubator at 30°C for thirty minutes, and dried again under reduced pressure by a vaccum dryer. The plate for fixing coated erythrocyte is thus obtained, which has erythrocyte coated in a single layer in its well bottoms and fixed by drying. According to this method, the coated cells can be fixed with activation of specific substance maintained.

EP 0 542 301 A1

This invention relates to a method of fixing coated biological substances, by coating biological substances containing tissues or cells, which are utilized for immunological reactions, and drying them.

Conventional immunological reactions utilizing coated tissues and cells are known. For example, in Japanese Laid – Open Patent Application No. 124464/90, a method of coating erythrocytes on a support such as a microplate well, a test tube, and a slide glass are described, and in International Archives of Allergy and Applied Immunology (Shibata, et. al, 74, 93 – 96, 1984), a method of coating platelets are described. These immunological reactions are applied, for example, to blood typing (ABO, Rh) which is required for a safe transfusion. They are also utilized for specific reactions between specific substances on a cell such as blood group substances, HLA, and platelet antigens, and their reactive substances which specifically react with them. The specific reactions provides numerous utility informations.

However, since the coated tissues or cells for said immunological reactions are live and its life span are very short, they cannot be preserved for a long period, and their effective periods are restricted. For example, in said Japanese Laid – Open Patent Application No. 12464/90, the coated erythrocytes cannot be preserved for a long period because they are live. Even if the erythrocytes have been suspended in e.g., Alserver solution or MAP solution after they are separated for preserving for a long period, the erythrocytes are hemolyzed immediately after they are separated. Therefore, the coated erythrocytes get useless as a reagent in several weeks to one month. Even O – type red cells for screening which are available from such as Ortho, Diagnostic Systems, Inc. cannot be preserved for a long period, and their effective period is set.

In order to lengthen their effective period, it is considered to coat the tissues and cells. Several methods of fixing tissues or cells are provided in e.g., cytodiagnosis and the like. A general method of the fixing for cytodiagnosis is to make proteins in the tissues or cells denaturative, e.g., the proteins are made to be denatured by utilizing solvents such as alcohol, heavy metals such as dichroic acid, fixative agents such as formalin and glutaraldehyde, as described in Manual of Cytotechnology. Japanese Patent Publica – tion No. 6941/86 also describes a method of fixing erythrocytes. Thus, other method of fixing include boiling or lyophilizing the tissues or cells.

According to said methods of fixing tissues or cells, they can be morphologically preserved and can be successfully stained by denaturing the proteins therein. However, these methods cannot be applied when fixing the tissues or cells for immunological reactions, because specific substances on the cells are denatured. This disadvantage is disclosed in Japanese Laid – Open Patent Application No. 151765/90, (pp. 383, the forth line on the left lower side – pp. 384, the forth line on the left lower side).

In order for specific substances not to denature, other methods of fixing tissues or cells are considered. According to these methods, tissues or cells are fixed by drying. A known method, for example, is to lyophilize artificial or natural particles combined with antigens such as viruses and bacteria, or with antibodies. Also, in USP 5030560, Immucor. Inc. provides a method of fixing animal tissues or cells coated on a support by drying.

However, the method described in said USP 5030560 (hereinafter abbreviated as the literature 1) suffers from the following disadvantage. As described in column 4, lines 44 – 47 and column 5, lines 47 – 50 in the literature 1, a single layer of cells such as erythrocytes and platelets is formed on a coating support which has already been stained with organic stains charged with net positive charges, and this coating is inhibited by proteins. Therefore, the cells should not have proteins adhered on their surfaces, etc., and a drying solvent utilized after coating is also required to be free of proteins. When electrically charged chemical substances such as cation dyes and poly – L – lysin of high concentrations is utilized, the substances liberate and adhere to the surfaces of the cells and make the cells to absorb antigens or antibodies which are contained in a test sample but are not target, or proteins which are contained in solutions utilized as additive, leading to inactivate selective reactivity of the cells. The optimum concentra – tion rages are thus restricted narrow, and a sufficient coating effect cannot be obtained. For this reason, the cells are required to wash, e.g., by centrifugation to remove proteins. However, even if the cells are suspended in a protein – free solution, proteins are liberated from the cells themselves gradually. For example, Japanese Journal of Transfusion (vol. 36, No. 2) describes antigens can be extracted only by suspending cells in a physiological saline. In this case, the cells must be washed again. As a conclusion, the method in the literature 1 requires complicated steps to remove proteins.

Separately, according to the literature 1, cells are dried again after they are fixed by drying with a drying solution. The literature 1, pp. 387, the 11th line of the left upper side to the second line on the right upper side, describes that it is preferable to slowly dry cells for a long time with molecularsieve desiccants, because sufficient drying cannot be obtained by general methods utilizing vacuum drying, or drying with diatomaceous earth or silica particles. However, this method is inappropriate to industrially fixation a large amount of cells by drying, because molecular – sieve desiccants are relatively expensive, and it takes much time to completion of drying. Therefore, a novel method is expected to develop, which enables cells to stably fix at a lower coat by vacuum drying or natural drying.

According to the method of fixing coated cells by drying described in the literature 1, proteins should be completely removed. However, the following problems are raised when coated cells are fixed in the absence of proteins.

First, in general, protein is necessary to avoid inactivation of reactivity and specificity of antigens or antibodies when they are preserved, it cannot be added. Thus, for example, when lyophilizing particles sensitized with antigens or antibodies, proteins such as bovine serum albumin (hereinafter abbreviated as BSA) and animal serum are generally added to a drying solution, in addition to sugar. More especially, Japanese Patent Publication No. 30668/90 described Table 1 (pp. 180) which lists compositions of drying solutions are their sensitivities (reactivities). The Table 1 shows that higher reactivity can be obtained when utilizing drying solutions added with proteins such as BSA. Japanese Laid – Open Patent Application No. 209362/87 describes a method of manufacturing sensitized corpsules liophilized by utilizing a drying solution which comprises suggar into an adequate solvent added serum albumin. In most of lists of mediums for lyophilizing particles sensitized with antigens or antibodies which can be available, it is shown that the mediums are added with proteins such as BSA and animal serum. However, according to the method of fixing coated cells by drying described in the literature 1, a drying solution added with proteins cannot be utilized, and thus inactivation of specific substances cannot be avoided.

Second, when coated cells are utilized for immunological reactions such as enzyme immunoassay, blocking cannot be performed without using proteins. Blocking is routinely performed to avoid non – specific reactions (reactions which are not specific and are not derived from the objective proteins) made by proteins. Therefore, if immunological reactions are made by utilizing erythrocytes prepared according to the method in the literature 1, in which blocking can not be performed, non – specific reactions are likely to occur.

Under the considerations, the object of the invention is to provide a method of fixing coated cells by drying, which can successfully fix the coated cells and avoid inactivation of specific substances.

The present invention provides a method of fixing coated biological substances by fixing biological substances having immunological activities to a support for analyzing the immunological reactivities refer to the biological substances, comprising; a step of applying a binder to the support, the binder adhere to the support and makes antigen/antibody reaction with the biological substances, a step of making the applied binder to contact with the biological substances, thereby coating the biological substance on the support, a step of making the coated biological substances to contact with a drying solution containing an effective amount of proteins which do not combine with the binder and blocks non – specific reaction of the biological substances, and a step of drying the biological substances which are made to contact with the drying solution to remove water, thereby fixing the biological substances.

Detailed description of the invention are as follows.

According to the invention, a drying solution utilized to fix coated biological substances by drying preferably contains sugars and peptides or proteins. Examples of the sugars are monosaccharides such as glucose and lactose, disaccharides such as saccharose and maltose, trisaccharides, and polysaccharides such as dextran. Among them, saccharose is most preferable. Concentration of the sugars is preferably 0.1 to 50 wt%.

Peptides or proteins are also added to avoid inactivation of specific substances in the drying solution. Examples of the peptides are carrier proteins and carrier peptides such as BSA, gelatin, and gelatin hydrolysates.

More especially, in order to avoid unnecessary immunological reactivities of the cells, the peptides or proteins are added to the drying solution as additive, which do not combine with the binder and blocks non – specific reactivities of biological substances. For example, BSA and gelatin are useful, because they show no immunological activity and do not combine with lectin.

The drying solution also contains adequate solvents such as water and physiological saline.

The drying solution most preferably contains 20 wt% of saccharose and 0.2 wt% of BSA.

Salts may be further added to the drying solution in order to adjust its osmotic pressure. Preferable examples of the salts are sodium chloride, phosphates, and pottasium chloride. However, other salts may be used.

According to the method of the present invention, lectin or antibodies are preferably utilized to coat tissues or cells on a support as binder. Examples of lectin are wheat germ lectin (hereinafter abbreviated as WGA), ricin (Rechnus communls), and lentil lectin. For example, lectin is utilized as a binder to coat a single layer of cells.

Since the fixed antigens or antibodies according to the present invention can be maintain their immunological activities on a support for a long period, they are useful for various kinds of immunological tests in diagnosis, culture, etc.

A shape of a support is not restricted to embody the present invention, and any shape can be utilized as far as it is appropriate for the object of the invention. For example, the cells can be coated to an inner wall of a flow tube, fiber surfaces, a sphere surface, a slide plate, a filter, thread surfaces, etc. Therefore, they are useful for various kinds of immunological tests.

Preferable examples of a support to coat tissues or cells are also wells of a microplate, a glass tube, and a slide glass.

The cells after fixed can be basically preserved for a long period, at any temperature as far as proteins do not denature. For example, 40°C or less is preferable, and 10°C or less is more preferable.

In additional, a biological substance may have a immunological activities, contains cells and tissues having specific substances on their surface, which can react with a determined antigen or antibody. Example of the biological substance are a erythrocyte, a platelet, a lymphocte, or the like.

According to the method of the present invention, tissues or cells are dried for fixing simultaneously or after they are coated on a support, by making the drying solution to contact with them. Then, the fixed tissues or cells are subjected to centrifugation to remove water. Thereafter, they are further dried with air in an incubator at 37°C, or completely drying by vacuum drying or lyophilization.

Fixing of the tissues or cells is also performed by directly suspending them in the drying solution. Although this method is not completely satisfactory, the coating of the cells on the substrate can be made simultaneously with fixing by the drying solution.

Also, in order to activity of specific substances can be maintained, the tissues or cells can be made to contact with a hypotonic solution to make their contents to elute (e.g. hemolysis), and they may be then made to contact with the drying solution.

The method of fixing coated biological substances according to the present invention may be applied to coat and fix such as erythrocytes, leucocytes, platelets, etc. which have specific substances such as antigens on their surfaces, etc, to a support substances.

The coated and fixed cells produced according to the present invention can be applied to immunological reactions such as enzyme immunoassay (EIA), radio immunoassay (RIA), and fluorescent antibody method, which utilize magnetic particles sensitized with antibodies or enzymes, as described in Japanese Laid-Open Patent Application No. 124464/90. Specifically, the coated and fixed cells are appropriate for assay systems utilizing magnetic particles.

According to the method of the present invention, the tissues or cells are coated by utilizing the binder which can adhere to the support and makes antigen/antibody reaction with the biological substances, e.g., lectin or antibodies. Therefore, they can be coated on the substrate without the abstraction with proteins eluted from themselves. Also, inactivation of specific substances of the coated tissues or cells are completely avoided during fixing, by making them to contact with a drying solution containing proteins. Therefore, when utilizing the coated cells for immunological reactions, non-specific reactions can be decreased by blocking with using the proteins. Further, the tissues or cells can be stably preserved for a long period by fixing them by drying. Therefore, a time required for a laboratory test utilizing immunological reactions can be largely decreased, and the effective period of the coated tissues or cells can be lengthened. Specifically, the method according to the present invention is advantageous for antibody identification, which has conventionally required fresh erythrocytes of as many as ten and several kinds, many reagents and manipulations, and much time. Further, prompt antibody identification of lower cost is possible by fixing panel erythrocytes of different antigenicities on each well.

This invention can be more fully understood from the following detailed description when taken in conjunction with the accompanying drawings, in which:

Fig. 1 is a photograph showing an image of erythrocytes which are fixing according to the method of fixing of coated cells of the present invention; and

Fig. 2 is a photograph showing an image of erythrocytes which are fixing after they are hemolyzed according to the method of fixing of coated cells of the present invention.

Detailed description of the preferred embodiments of the present inventions is as follows.

Embodiment 1

1-1. Production of a microplate for coating erythrocytes

To a U-bottomed plate (Maxisorp type, manufactured by Nunc, Inc.), WGA of 10 μg/mℓ in 0.01M phosphoric acid buffer solution (hereinafter abbreviated as PBS) (Ph 7.0) was dispensed to each well in 50 μℓ portions. Then, the plate was incubated at room temperature for thirty minutes, and washed with PBS five times to obtain a microplate for coating erythrocytes, which had wells coated with WGA.

1 – 2. Coating of erythrocytes

O – type red cell Surgiscreen (registered trade mark) 1 for screening of known antigenicity (manufactured by Ortho Diagnostic Systems, Inc.) was diluted with PBS to adjust a concentration of the red cells to ca.1.0 wt% for suspension, dispensed in 25 micro ℓ portions to each well of said microplate for coating erythrocytes obtained in 1 – 1, and allowed to stand at room temperature for ten minutes. Next, each well was washed with physiological saline three times to prepare a plate for coating erythrocytes, which has a single layer of erythrocytes formed on its well bottoms.

1 – 3. Drying of erythrocytes

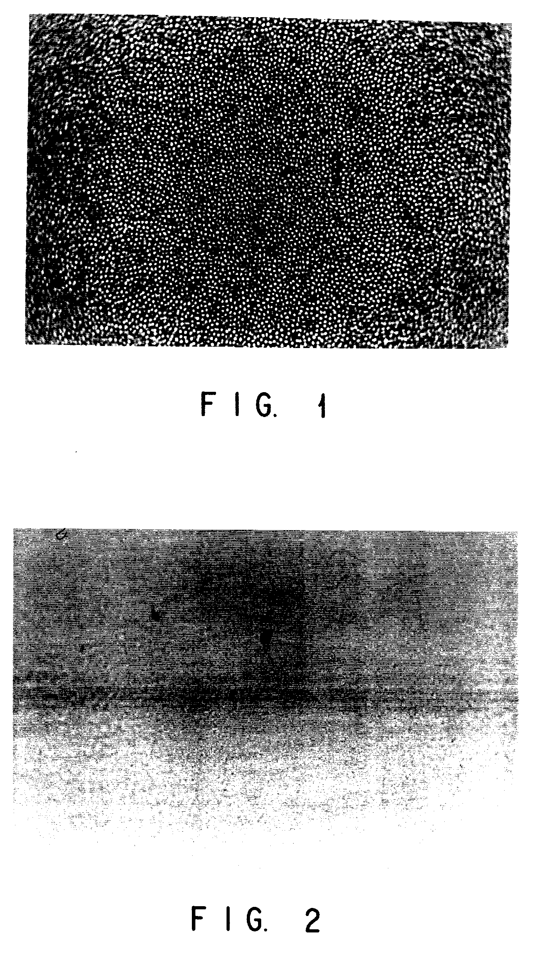

To the plate for coating erythrocytes prepared in 1 – 2, a drying solution containing 20 wt% of saccharose, 0.2 wt% of BSA, and 0.05 wt% of sodium dithionite was dispensed to each wells of the plate in 100 $\mu\ell$ portions. Next, the plate was allowed to stand for three minutes, and the drying solution was discarded and water was removed. The plate was then air – dried by a dry incubator at 30°C for thirty minutes, and further dried under reduced pressure by a vacuum dryer for four hours to prepare a plate for fixing dried erythrocytes (hereinafter described as dried erthyrocyte – fixed plate). Each well of the plate is coated with dried erythrocytes on its bottom, and looked light brown. Fig. 1 is a microphotograph showing an image of the erythrocytes. As apparent from Fig. 1, the erythrocytes are fixed in a single layer with their morphology maintained.

1 – 4. Detection of antibodies

To each well of the dried erythrocyte – fixed plate prepared in 1 – 3, LISS (lower – ion isotonic solution) was dispensed in 25 $\mu\ell$ portions. Therefore, the dried erythrocytes got wet, returned to their original conditions, and hemolyzed. The image of erythrocytes got unseen. Next, anti – D serum and another serum derived from healthy and normal people having no antibodies were dispensed to pre – determined wells, respectively. The plate was then incubated at 37°C for ten minutes, and each well was washed with physiological saline six times. Thereafter, a suspension of magnetic particles sensitized with anti – human IgG (manufactured by Olympus Optical Co., Ltd.) was dispensed in 25 $\mu\ell$ portions to each well. The plate was mounted on a magnet for three minutes to form a pattern. For control, a plate for fixing erythrocytes without drying (hereinafter described as non – dried erthrocyte – fixed plate) was prepared by subjecting erythrocytes to the same treatments as said, with the exception that they were not fixed by the drying solution.

As sated above, assay was performed with anit – D serum by utilizing the dried erythrocyte – fixed plate and the non – dried erythrocytes – fixed plate. Reactivity of anti – D serum for each plate was evaluated as shown in Table 1.

Table 1

| Plate | Dilution of anti D – serum | | | | | | | Negative serum |
|---|---|---|---|---|---|---|---|---|
| | 100 | 200 | 400 | 800 | 1600 | 3200 | 6400 | 12800 |
| Dried | + + | + + | + + | + + | ± | ± | – | – – |
| Not dried | + + | + + | + + | + + | ± | – | – | – – |

*+ +: a pattern is uniformly spread on a whole bottom of a well
+: a pattern is likely to contract with its external circumference broken
–: a pattern concentrates to the center of the well and looks like a button
+: a pattern spreading to a medium space between + and – patterns

As apparent from the Table 1, immunological reactivity of anti – D serum in the dried erythrocytes – fixed plate was found to be as high as that of the non – dried erythrocytes – fixed plate. As a conclusion, antigenicity was not lowered.

## Embodiment 2

According to the same manner as described in the embodiment 1, an assay was performed by using the same dried erythrocytes–fixed plate as prepared in the embodiment 1 to eight kinds of utilizing antiserums (manufactured by Ortho Diagnostic Systems Inc. and by BCA Inc.) with an exception that $Le^a$ is serum derived from a patient, in order to detect erythrocyte antigens and evaluate reactivity of each antiserum. The result is shown in Table 2.

Table 2

| | Antiserum | | | | | | | | Negative serum |
|---|---|---|---|---|---|---|---|---|---|
| | D | E | c | k | Fyb | Jk$^a$ | Le$^a$ | s | |
| Antigen in erythrocytes | pre-sent | pre-sent | pre-sent | pre-sent | pre-sent | pre-sent | pre-sent | pre-sent | |
| Reactivity | ++ | ++ | ++ | ++ | ++ | ++ | ++ | ++ | − |

\* ++ and − indicate the same as in the Table 1

As apparent from the Table 2, every erythrocyte antigen shows antigenicity. It is noted that $Le^a$ shows antigenicity, which is an adsorption antigen.

## Embodiment 3

By utilizing binder such as Ricin, lentil lectin, and polyethylenimine (PEI) other than WGA, a dried erythrocytes–fixed plate was prepared. Namely, Ricin and lentil lectin were dissolved in PBS of 10 μg/mℓ, and dispensed to each well of U–bottomed plates (manufactured by Nunc, Inc.), which were then incubated at room temperature for thirty minutes.

6

Separately, PEI (manufactured by Nacalai Tesque, Inc.) was diluted to prepare a solution of 0.01 wt%, and dispensed to each well of the U−bottomed plate (manufactured by Nunc, Inc.) in 50 $\mu\ell$ portions, and the plate was then incubated at room temperature for thirty minutes.

Next, to the plates coated with Ricin, lentil lectin, and PEI on their well bottoms, O−type erythrocytes for screening of known antigenicity were coated and then fixed by drying according to the same manner as described in the embodiment 1. Each plate thus prepared was utilized to assay with anti−D serum and negative serum, and reactivity of each serum was evaluated. The result was shown in Table 3.

Table 3

| Binder | Anti−D serum | Negative serum |
|---|---|---|
| Ricin | + + | − |
| Lentil lectin | + + | − |
| PEI | + + | − |

\* + + and − indicate the same as in the Table 1

As apparent from the Table 3, the binders other than WGA were confirmed to be successfully utilized, with a slightly different negative pattern.

Embodiment 4

Here is described a method of lyophilizing erythrocytes after they are coated and dried for fixing with a drying solution.

To the plate for coating erythrocyte prepared in 1−2 of the embodiment 1, Erythrocyte Sergiscreen (manufactured by Ortho Diagnostic Systems, Inc.) was coated. Next, a drying solution containing saccharose of 20 wt%, BSA of 0.2 wt%, and sodium dithionite of 0.05 wt% was dispensed in 100 $\mu\ell$ portions to each well of the plate and was allowed to stand for three minutes. The drying solution was then discarded and water was removed. The plate was transferred to a lyophilizing dryer, i.e., Stoppering Tray Dryer (manufactured by Labconco, Inc.), the plate had been cooled to −40°C, lyophilized to −40°C, and allowed to stand at −20°C for ca. twenty hours under reduced pressure, thereby performing the lyophilization of the erythrocytes.

The plate thus prepared was utilized to assay with anti−D serum and negative serum. The result was that reactivity coupled be apparently discriminated between positive and negative, as that in the embodiment 1.

Embodiment 5

Here is described a case of fixing of a erythrocyte by drying, which was made to elute contents of the erythrocyte with its morphology maintained, i.e., erythrocyte ghost.

First, to each well of the plate for coating erythrocyte prepared in 1−2 of the embodiment 1, erythrocytes were coated according to the same manner as said in the embodiment 1. Next, distilled water was dispensed to each well of the plate in 100 $\mu\ell$ portions, and the plate was allowed to stand for one minute to hemolyze the erythrocytes. After the distilled water was discarded, one of drying solutions a−f shown in Table 4 to each well of the plates, and was allowed to stand for three minutes. The drying solutions were then discarded and water was removed. The erythrocytes were then dried for fixing according to the same manner as said in the embodiment 1 to prepare a dried erythrocyte−fixed plate.

The plate thus prepared was utilized to assay with anti−D serum and negative serum according to the same manner as said in the embodiment 1. Reactivity was evaluated, and the result was shown in Table 4.

7

Table 4

| | | Drying Solution | Anti − D serum | Negative serum |
|---|---|---|---|---|
| a | | 20 wt% saccharose, 0.2 wt% BSA | + + * | failure ** |
| b | | 10 wt% saccharose, 0.2 wt% BSA | + + | failure |
| c | | 5 wt% saccharose, 0.2 wt% BSA | + + | − − |
| d | | 2.5 wt% saccharose, 0.2 wt% BSA | + + | − − |
| e | | 1.25 wt% saccharose, 0.2 wt% BSA | + + | − − |
| f | | 0.05M PB, 0.45 wt%NaCℓ, 2.5 wt% saccharose, 0.2 wt% BSA | + + | − − |

\* + +, − show the same as said in the Table 1
\*\* a pattern having its contour blotted

As apparent from the Table 4, the drying solutions (c − f), whoes osmotic pressures are lower than that of the erythrocytes were found to be effective in order to fix hemolyzed erythrocytes by drying them.

Fig. 2 is a microphotograph showing an image of erythrocytes which were hemolyzed, and fixed to the well bottom by drying. As apparent from Fig. 2, hemolyzed erythrocyte was different from that in the Fig. 1. Thus, the erythrocytes were morphologically broken, coated, and dried for fixing in ghost condition.

Embodiment 6

As shown in Table 5, drying solutions I − VII containing saccharose of varied concentrations, VIII and XI containing no BSA, and X and IX containing lactose or glucose instead of saccharose were prepared. By utilizing these drying solutions, dried erythrocyte − fixed plates were prepared according to the same manner as said in the embodiment 1. The plates were utilized to assay with anti − D serum and negative serum. Reactivity was evaluated, and the result was shown in the Table 5.

Table 5

| | | Drying Solution | Anti − D Serum | Negative Serum |
|---|---|---|---|---|
| I | | 40 wt% saccharose, 0.2 wt%BSA | + + | − |
| II | | 20 wt% saccharose, 0.2 wt%BSA | + + | − |
| III | | 10 wt% saccharose, 0.2 wt%BSA | + + | − |
| IV | | 5 wt% saccharose, 0.2 wt%BSA | + + | − |
| V | | 2.5 wt% saccharose, 0.2 wt%BSA (partly hemolysis) | + + | failure ** |
| VI | | 1.25 wt% saccharose, 0.2 wt%BSA (hemolysis) | + + | failure ** |
| VII | | 0.05M PBS.0.45 wt%NaCℓ 2.5 wt% saccharose, 0.2 wt%BSA | + + | − |
| VIII | | 5 wt% saccharose | + + | failure |
| XI | | 10 wt% saccharose | + + | failure |
| X | | 20 wt% lactose, 0.2 wt%BSA | + + | − |
| IX | | 20 wt% glucose, 0.2 wt%BSA | + + | − |

\* + +, − show the same as said in the Table 1
\*\* a pattern having its contour blotted

As apparent form the Table 5, when erythrocyte is fixed by drying without hemolysis, the drying solutions I − IV, X, and XI were found to be appropriate to use, in order to avoiding to hemolyze the erythrocyte. These solutions contain both BSA and one of saccharose, lactose, or glucose, and their concentrations are adjusted to make their osmotic pressures to be as high as, or higher than that of erythrocyte.

Embodiment 7

Here is described a method of fixing by suspending erythrocytes in a drying solution. First, to a drying solution containing 0.05M PBS, sodium chloride of 0.45 wt%, saccharose of 2.5 wt%, and BSA of 0.2 wt%, Red Cell Surgiscreen Cell (Rh + ) (manufactured by Ortho Diagnostic System, Inc.) was added to prepared a suspension of 0.7 wt%. This erythrocyte suspension was dispensed in 25 μℓ portions to each well of the plate for coating erythrocyte prepared according to the same manner as said in 1 − 2 of the embodiment 1. The plate was then allowed to stand at room temperature for ten minutes. The drying solution was then discarded with erythrocytes which are not coated to the plate. Next, the coated erythrocytes were fixed according to the same manner as said in the embodiment 1 to prepare a dried erythrocyte − fixed plate. This plate was utilized to assay with anti − D serum and negative serum. The result was that reactivity can be apparently discriminated between positive and negative as that in the embodiment 1.

Embodiment 8

A method of fixing platelets by drying them

The blood was taken from a subject who is positive for platelet PLA[1], added with ACD, and centrifuged for ten minutes to prepare platelet − rich plasma (PRP). To the PRP, ACD solution of 10 wt% relative to the PRP was added, and the centrifuged at 1100G for fifteen minutes to obtain platelet concentrate (PC). The PC was then washed with physiological saline twice. Then a platelet solution was prepared to become its concentration of ca. $10,0000/\mu\ell$, and dispensed in $25\ \mu\ell$ portions to each well of a microplate which has WGA coated on its well bottoms in the same manner as said in 1 − 2 of the embodiment 1. The microplate was centrifuged at 650G for five minutes to coat platelets on the well bottoms.

Next, chloroquine solution was dispensed to each well of the plate in $50\ \mu\ell$ portions, and the plate was allowed to stand at room temperature for thirty minutes. After chloroquine solution was discarded, the plate was washed with physiological saline three times.

To each well, a drying solution containing saccharose of 20 wt% and BSA of 0.2 wt% was then dispensed in $100\ \mu\ell$ portions. After the plate was allowed to stand for three minutes. Thereafter, the drying solution was discarded and water was then removed. A plate for coating dried platelet was thus prepared according to the same manner as said in the embodiment 1 except for using the platelet replace with the erythrocyte.

This plate was utilized to assay with PLA[1] − positive and negative controls of anti − PLT − olibio − MPHA (trade name) (manufactured by Olympus Optical Co., Ltd). The result was that reactivity can be apparently discriminated between positive and negative.

Experimental embodiment

The drying solution II in the embodiment 6 as representation was utilized for fixing, and the detection sensitivity was measured for comparison with the conventional method which coats tissues or cells to a reaction vessel by absorptivity of electrically charged substances.

In detail, a microtitre plate was prepared by fixing the erythrocytes according to the same manner as said in the embodiment 6, with the exception that polyethylenimine (M.W. = 60,000 − 80,000) was utilized instead of lectin (hereinafter in described as conventional embodiment), and utilized to detect anti − D antibody for comparison with the plate which was prepared by utilizing the drying solution II in the embodiment 6. As the result, in the conventional embodiment, which fix the erythrocytes by absorptivity of electrically charged substances, although the same detection sensitivity was obtained as that in the plate for the embodiment 6, an agglutination pattern of high concentration is not apparently discriminated with a non − agglutination one when a concentration of the solution was high than a utility concentration range for fixing, i.e. more 0.05 wt%. Therefore, according to the conventional embodiment, it was very difficult to decide between the agglutination pattern and the non − agglutination one. It can be thought that this is because the charges derived from polyethylenimine on the well surface make the proteins in the drying solution or the sample to combine with the cells coated on the well in non − specific manner, leading to inactivation of the specific immunological reactivity. Therefore, electrically charged substances cannot be utilized, since the phenomena caused by them as described above often makes the decision to be error.

On the other hand, in the method which applies the embodiment 6 according to the invention, the agglutination pattern can be easily discriminated with the non − agglutination one, and the decision was also easy. Therefore, since the cells for fixing can be dissolved in the drying solution containing said proteins, made to contact with the binders on a support, and dried without replacing the solution, with specific immunological reactivity maintained, the present invention is advantageous in that it is economical and requires only few steps.

Further, in this embodiment when corpusclar cells for fixing were fixed in hypotonic condition, necessary immunological reactivity of the dried cells in the final step was found to be the same as that when the cells were not dissolved. This means that a step of removing unnecessary contents from the cells can be performed not only after coating the cells to a support but also simultaneously with a step of coating. Therefore, this also means an efficient preparation is thus possible by the present invention.

Furthermore, the plate according to the invention was preserved at 10°C for more than four weeks, and was found to provide the same detection sensitivity as that provided before preservation.

**Claims**

1. A method of fixing coated biological substances by fixing biological substances having immunological activities to a support for analyzing the immunological reactivities refer to said biological substances, comprising;

    a step of applying a binder to the support, said binder can adhere to the support and makes antigen/antibody reaction with said biological substances,

    a step of making the applied binder to contact with the biological substances, thereby coating the biological substance on the support,

    a step of making the coated biological substances to contact with a drying solution containing an effective amount of proteins which do not combine with the binder and blocks non-specific reaction of the biological substances, and

    a step of drying the biological substances which are made to contact with the drying solution to remove water thereby fixing the biological substances.

2. The method according to claim 1, characterized in that the binder can adhere to the support in a liquid phase.

3. The method according to the claim 2, characterized in that the binder is lectin.

4. The method according to the claim 3, characterized in that lectin is wheat germ lectin, ricin, or lentil lectin.

5. The method according to claim 2, characterized in that the binder is a substance substantially corresponded to antibody which is obtained by utilizing said biological substances as immunogen.

6. The method according to claim 1, characterized in that the drying solution contains sugars selected from a group consisting glucose, lectose, saccharose, maltose, trehalose, and dextran.

7. The method according to claim 1, characterized in that proteins contained in the drying solution are proteins derived from animal serum and/or gelatin.

8. The method according to the claim 1, characterized in that drying is performed by vacuum drying, natural drying, utilization of water-absorbable agent, or lyophilization.

9. The method according to claim 1, characterized in that the biological substances are tissues or cells derived from human beings.

10. The method according to the claim 9, characterized in that the biological substances are made to contact with a hypotonic solution at latest before drying to elute contents therein, and the drying solution, of which the osmotic pressure is adjusted to less than isotonic to the biological substances is used.

11. The method according to the claim 9, characterized in that the biological substances are coated and fixed to the support in an isotonic solution or in a hypertonic solution.

12. The method according to the claim 1, characterized in that the biological substances are erythrocytes, lymphocytes, or platelet cells.

13. The method according to the claim 9, characterized in that the biological substances are coated to form a single layer.

14. The method according to the claim 1, characterized in that the support is made to contact with a liquid phase, before analyzing the immunological reactivities of the biological substances.

15. The method according to the claim 14, characterized in that the liquid phase is a reagent or a sample.

16. The method of according to the claim 1, characterized in that the support is a reaction vessel to detect the biological substances by antigen/antibody reaction.

17. The method according to the claim 16, characterized in that the vessel is a microtitre plate, a glass tube, or a slide glass.

18. The method according to the claim 1, characterized by further comprising a step of preserving the support on which the biological substances are coated and fixed at an appropriate temperature.

F I G. 1

F I G. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US-A-5 030 560 (LYLE T. SINOR; RALPH A. EATZ) <br> * the whole document * <br> --- | 1-18 | G01N33/543 <br> G01N33/566 <br> G01N33/569 <br> G01N33/80 |
| Y | DATABASE WPIL <br> Section Ch, Week 8537, <br> Derwent Publications Ltd., London, GB; <br> Class A96, AN 85-226924 <br> & JP-A-60 147 650 (TOSHIBA KK) 3 August 1985 <br> * abstract * <br> --- | 1-18 | |
| P,Y | EP-A-0 469 766 (BECTON, DICKINSON & COMPANY) <br> * page 4, column 5, line 9 - line 36 * <br> * page 5, column 7, line 20 - column 8, line 46 * <br> * claims 1-10 * <br> --- | 1-18 | |
| D,A | DATABASE WPIL <br> Section Ch, Week 8742, <br> Derwent Publications Ltd., London, GB; <br> Class B04, AN 87-296561 <br> & JP-A-62 209 362 (KAGAKU OYOBI KESSEI) 14 September 1987 <br> * abstract * <br> --- | 1-18 | |
| A | DATABASE WPIL <br> Section Ch, Week 8431, <br> Derwent Publications Ltd., London, GB; <br> Class B04, AN 84-192447 <br> & JP-A-59 109 862 (TAKEDA CHEMICAL IND KK) 25 June 1984 <br> * abstract * <br> & JP-B-2 030 668 (category D) <br> --- | 1-18 | |
| A | EP-A-0 351 857 (OLYMPUS OPTICAL CO., LTD.) <br> * the whole document * <br> & JP-A- 2 124 464 (category D) <br> --- | 1-18 | |

TECHNICAL FIELDS SEARCHED (Int. Cl.5)

G01N
C12N

The present search report has been drawn up for all claims

| Place of search | Date of completion of the search | Examiner |
|---|---|---|
| THE HAGUE | 02 MARCH 1993 | DOEPFER K.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

EUROPEAN SEARCH REPORT

Application Number

EP    92 11 9465
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | DATABASE WPIL<br>Section Ch, Week 9031,<br>Derwent Publications Ltd., London, GB;<br>Class A89, AN 90-234526<br>& JP-A-2 161 357 (TOSOH CORP.) 21 June 1990<br>* abstract * | 1-18 | |
| A | DATABASE WPIL<br>Section Ch, Week 8436,<br>Derwent Publications Ltd., London, GB;<br>Class A96, AN 84-222249<br>& JP-A-59 130 178 (AMANO PHARM KK) 26 July 1984<br>* abstract * | 1-18 | |
| D,A | INTERNATIONAL ARCHIVES OF ALLERGY AND APPLIED IMMUNOLOGY<br>vol. 74, 1984, BASEL CH<br>pages 93 - 96<br>YOICHI SHIBATA ET AL. 'Mixed Passive Hemagglutination with Soluble Platelet Antigens'<br>* the whole document * | 1-18 | TECHNICAL FIELDS SEARCHED (Int. Cl.5 ) |
| A | WO-A-9 004 329 (COULTER CORPORATION)<br>* claims 1-22 * | 1-18 | |

The present search report has been drawn up for all claims

| Place of search | Date of completion of the search | Examiner |
|---|---|---|
| THE HAGUE | 02 MARCH 1993 | DOEPFER K.P. |

EPO FORM 1503 03.82 (P0401)